# EUROPEAN PATENT SPECIFICATION

(11) **EP 2 301 611 B1**
(45) Date of publication and mention of the grant of the patent: **15.08.2012**
(21) Application number: 09171370.1
(22) Date of filing: 25.09.2009
(51) Int. Cl.: A61M 5/145, A61M 5/20

(54) **Medical delivery device**
Medizinische Abgabevorrichtung
Dispositif d'administration médicale

(43) Date of publication of application: 30.03.2011
(73) Proprietor: Letcat Aktiebolag, 223 69 Lund (SE)
(72) Inventor: Landahl, Hendrik, 227 63, LUND (SE); Floreby, André, 245 45, STAFFANSTORP (SE)
(74) Representative: Bergström, Johan Erik

(56) References cited:
- WO-A1-01/72361
- US-A- 2 309 502
- US-A- 2 725 877
- US-A- 3 298 666
- US-A- 3 502 358
- US-A- 3 797 488
- US-A- 5 679 111

## Description

### TECHNICAL AREA

The present invention relates to a medical delivery device and in particular an injector for self-administration of medicament.

### TECHNICAL BACKGROUND

Medicament delivery devices specially designed and intended for primarily self-administration have been developed during a number of years and numerous different solutions have been invented for this purpose.

One major important factor when developing a medicament delivery device and in particular injectors is that they should be easy to use for a number of different user categories as well as reliable functionality regarding dose delivery.

Different developers have made different approaches to the ease of use criteria, whereby a lot of functions have been automatic in the sense that they are performed automatically after activation of the device These automatic functions may include mixing of medicament, priming of the medicament container, penetration, injection, withdrawal of the needle after injection as well as covering of the needle by needle shields.

Even though automatic functions may help a user, they may at the same time cause such a complex design of the delivery device that they actually make handling of the device more difficult and less intuitive. Also adding of functions leads to added number of components in the injector as well as added requirements on interaction and tolerances and the like in order for the automatic functions to really work and be performed in a reliable way. The added functionality also adds to the manufacturing cost of the delivery device.

A medicament delivery device according to the preamble of claim 1 is known from US 3 797 488 A.

There is thus still a demand for delivery devices that can provide a reliable and consistent functionality to a user in an intuitive manner without making the delivery device too complicated to use nor too complex regarding its design and functions.

### BRIEF DESCRIPTION OF THE INVENTION

The aim of the present invention is to provide a medicament delivery device that provides the user with a high degree of functionality and ease of handling yet not having excess numbers of components.

This aim is obtained by the features of the independent patent claims. Preferable embodiments of the present invention are found in the dependent patent claims.

According to claim 1, the invention comprises a medicament delivery device comprising a housing; a medicament container arranged inside said housing, a drive mechanism comprising a plunger rod capable of acting on a stopper inside said medicament container for expelling a dose of medicament, said drive mechanism further comprising a force member acting on said plunger rod, an actuation mechanism capable of releasing said drive mechanism upon activation, **characterised in that** said actuation mechanism comprises a locking mechanism capable of releasibly locking said plunger rod in any positions between an initial start position and an end position.

Moreover, said locking mechanism comprises a one-way locking unit enabling the plunger rod to move in only a proximal direction.

According to an embodiment of the invention, said one-way unit comprises rolls or balls in contact with said plunger rod and a locking member arranged with inclined surfaces acting on said rolls or balls. According to the invention, it comprises a further rod arranged generally parallel to said plunger rod and wherein said actuation mechanism acts on said further rod.

Preferably, said actuation mechanism is activated by a manually operated actuation button or alternatively by a needle shield arranged in the proximal end of said medicament delivery device.

It may further comprise a dose setting mechanism comprising a threaded dose setting member and wherein it further comprises second locking means for releasibly locking said threaded dose setting member.

Said second locking member may manually be activated to release said threaded dose setting member when a medicament container is to be placed in said medicament delivery device.

Preferably, it further comprises a lid, arranged to said housing for replacing said medicament container.

The present invention has a number of advantages in comparison with the known devices in this technical area. The use of a locking mechanism arranged to releasibly lock the plunger rod provides a freedom of operation that can not be obtained by conventional threaded plunger rods cooperating with nuts. The provision of the one-way lock with members pressed by inclined surfaces may ensure a very firm and stable locking where the locking force becomes stronger the higher force applied.

The locking mechanism acts directly on a second rod arranged generally parallel with the plunger rod. The solution with a second rod, a dose release brake rod placed generally parallel with the plunger rod provides a compact device which nonetheless is easily controllable since the full length of the dose release brake rod may be used for placing sensors as well as for controlling the movement. This is not readily possible with only a plunger rod since it moves into the medicament container during injection. A preferable locking device utilises rolls or balls that are pressed against the actuation rod, which provides a very positive locking ability without excessive number of components.

The activation could then be performed either by a manually operated button or by a needle shield arranged at the proximal end of the device, wherein pressing of the device against an injection site causes the needle shield to move and thus activate the medicament delivery.

Another preferable and advantageous feature of the present invention is the lid, whereby it is easy to replace a medicament container. In that respect the lid is utilized for moving the plunger rod such that it is out of the way when a new container is to be placed, and wherein the plunger rod is moved back by the lid and force means when the lid is closed.

In all a new and inventive design of a medicament delivery device is obtained with the present invention.

These and other aspects of and advantages with the present invention will become apparent from the following detailed description and the accompanying drawings.

### BRIEF DESCRIPTION OF THE DRAWINGS

In the detailed description, reference will be made to the accompanying drawings, of which
Fig. 1 is a schematic view of some preferred functions of the present invention,
Fig. 2 is a detailed schematic view of a principle dose release mechanism,
Fig. 3 is a detailed schematic view of an alternative placement of a dose release mechanism of Fig. 2,
Fig. 4 is a schematic detailed view of a cartridge exchange brake,
Figs. 5, 6 are schematic detailed views of alternative cartridge exchange brake solutions,
Fig. 7 is a schematic detailed view showing dose limits preferred with the present invention,
Fig. 8 is a perspective view of a first embodiment of a medicament delivery device arranged with an injection button according to the invention,
Fig. 9 is a perspective view of the medicament delivery device of Fig. 8 but opened for access of medicament container,
Fig. 10 is an exploded view of the medicament delivery device of Fig. 8,
Fig. 11 is a side view in cross-section of the medicament delivery device of Fig. 8,

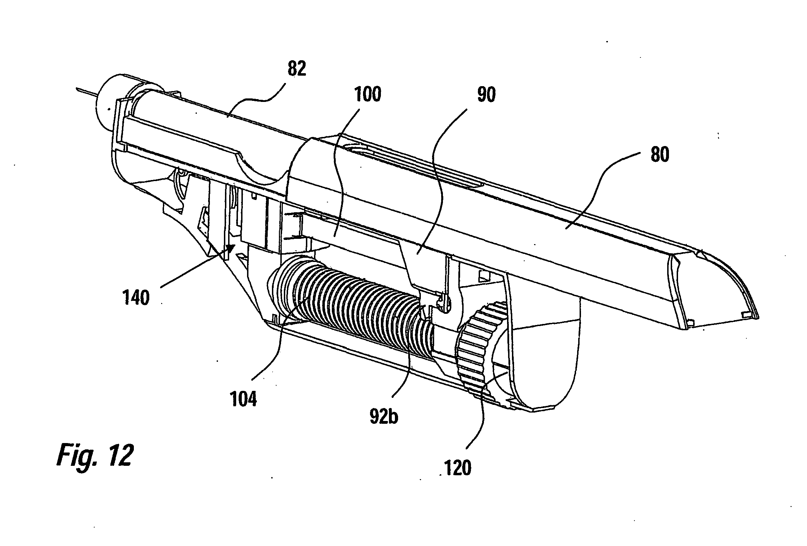

Fig. 12 is a perspective view of the device of Fig. 9 with part of the housing removed for clarity,
Fig. 13 is a detailed view taken along line XIII - XIII of Fig. 11 from above of a cartridge exchange mechanism comprised in the present invention,
Fig. 14 is a detailed view of dose release brake mechanism,
Fig. 15 is a further detailed view taken along line XV - XV of Fig. 11 from above of the dose release brake mechanism, and
Fig. 16 is a perspective view of a second embodiment of a medicament delivery device arranged with a needle shield also acting as an injection activator.

### DETAILED DESCRIPTION OF THE INVENTION

In the present application, when the term "distal part/end" is used, this refers to the part/end of the delivery device, or the parts/ends of the members thereof, which under use of the delivery device, are located the furthest away from the medicament delivery site of the patient. Correspondingly, when the term "proximal part/end" is used, this refers to the part/end of the delivery device, or the parts/ends of the members thereof, which under use of the delivery device, are located closest to the medicament delivery site of the patient.

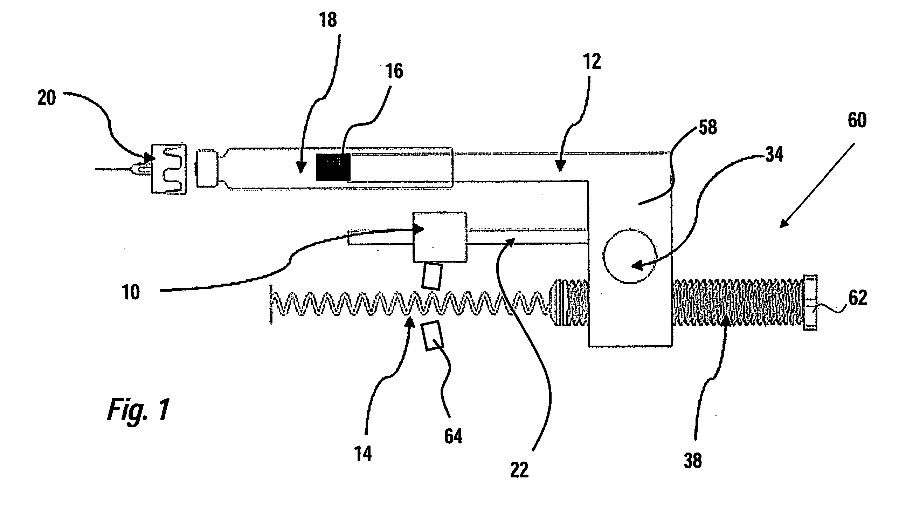

Figures 1 to 7 of the drawings display different important or key features comprised in the present invention. In order to highlight these features other support components or other functions of a medicament delivery device have been removed. Other relevant components and/or functions will become apparent and will be described in a non-limiting example of a medicament delivery device comprising the present invention.

Figure 1 shows a schematic view of some important basic components and functions according to the present invention as will be described.

### Dose release brake

One important feature is a locking unit, hereafter named dose release brake 10. It is designed as a locking unit that is capable of locking and releasing a plunger rod 12, which plunger rod 12 is urged in the proximal direction by a loaded medicament delivery force unit 14, such as e.g. a spring, a gas cylinder and the like. The plunger rod 12 is urged against a movable stopper 16 arranged inside a medicament container 18. A medicament delivery member 20 may be attached to the medicament container 18, such as an injection needle as shown. It is however to be understood that other types of medicament delivery members may be utilized such as mouth or nose nozzles, aerosol creating membranes, nebulizers and the like.

The dose release brake 10 is designed such that it may be locked and released in any position from an initial position to an end position. The locking unit may be acting either directly on the plunger rod 12 or on a separate rod 22, hereafter named dose release brake rod, fixed in relation to the plunger rod and arranged generally parallel with the plunger rod.

The general function is that the dose release brake 10 will keep the dose release brake rod 22 and thus the plunger rod 12 fixed while the user is setting a dose, as will be described. When a medicament delivery is performed the dose release brake 10 is opened by an appropriate release mechanism such as an injection button and the set dose of the drug is delivered from the medicament container 18. According to one example of the dose release brake 10, it should allow movement in one direction only and could be designed such that the higher the force applied, the harder the braking force The function of the self-locking dose release brake 10 is such that the user may interrupt the injection by releasing the button.

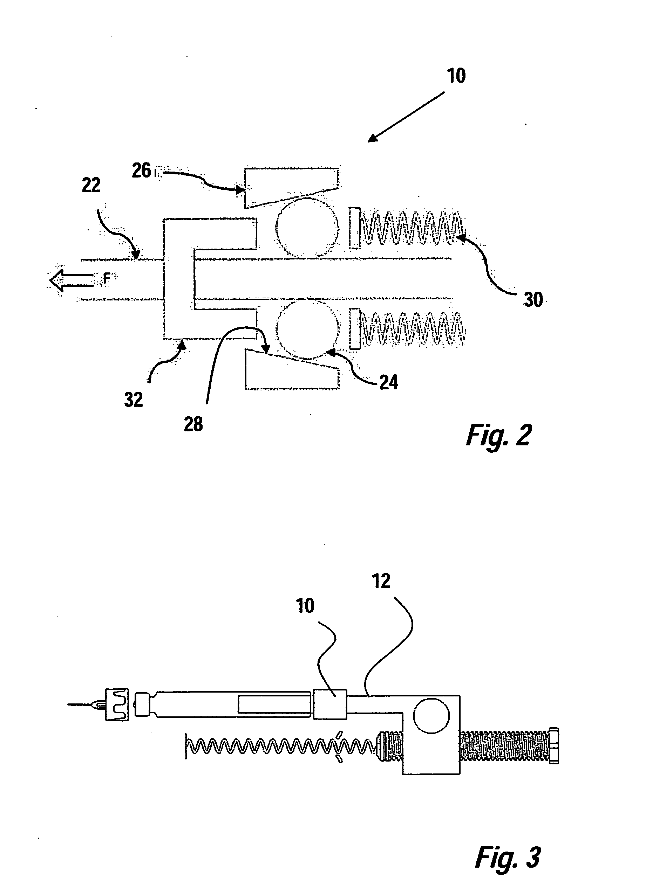

Figure 2 shows a schematic view of a possible self-locking function of the dose release brake 10. The dose release brake rod 22 is kept fixed/locked by locking members 24 such as cylindrical pins, rolls or possible balls. These are pressed between two pressing members 26 having inclined surfaces 28 and the rod 22. As an addition, in order to avoid slip, when no or a minimum of load is applied the locking members 24 are kept in place by a spring element 30. When a medicament delivery shall be performed, a release member 32 pushes the locking members 24 and the dose release brake rod 22 is able to slide.

As an alternative, as seen in Fig. 3, the dose release brake 10 may be arranged directly on the plunger rod 12, thereby avoiding the need for a separate dose release brake rod.

### Cartridge exchange brake

Another important feature is a cartridge exchange brake 34. The function of the cartridge exchange brake is to allow the plunger rod 12 to be moved in the distal direction when a used medicament container 18 is to be replaced by a new container, such that the plunger rod is disconnected from the medicament delivery force unit 14 during the exchange. After the exchange, the cartridge exchange brake 34 again locks the plunger rod operationally to the medicament delivery force unit 14.

The cartridge exchange brake 34 may be designed in several ways. Three different locking methods are described below, two designs where the locking method is by pure friction and one design using splines. The design choice is dependent on robustness requirements, where splines is considered to be more robust however lacking the same locking resolution as the friction method.

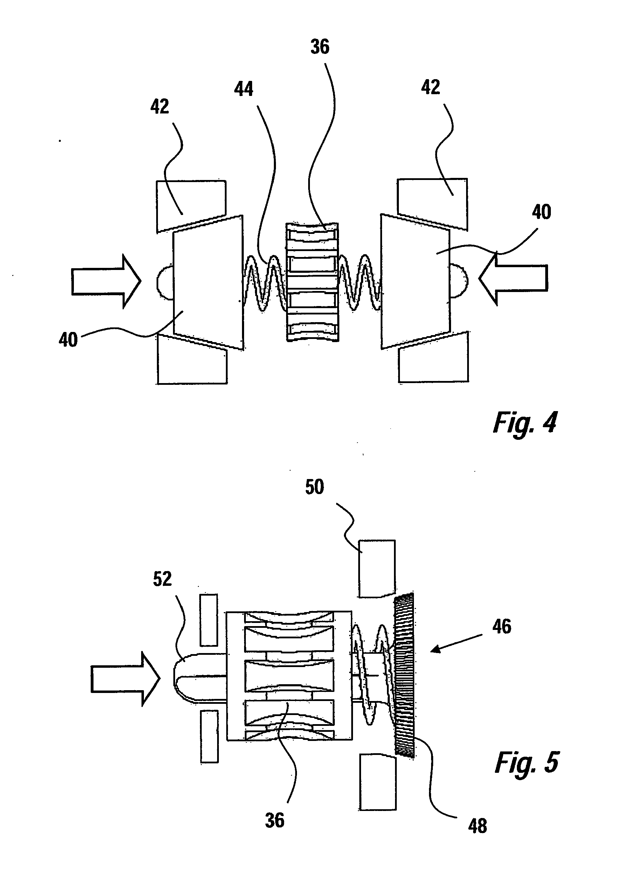

The technical function of a double-sided friction brake is shown in figure 4. A worm wheel 36 locks the linear movement of a dose setting screw 38, which will be described in detail below. It is kept locked in a rotational way by two friction cones 40 that lock by friction in a respective friction seat 42. The worm wheel 36 and the friction cones 40 are linked to each other in a non-rotational way. The force that keeps the worm wheel 36 locked is performed by springs 44. The cartridge exchange brake 36 could also be designed in reverse way, such that the cones (using splines) have a negative angle, in combination with a spring that works the opposite way.

During a cartridge exchange the medicament delivery device will be reset and the plunger rod 12 has to go to the far back position to be able to take the cartridge out/in. By pressing the two friction cones 40 inwards according to the arrows, the springs 44 will be compressed and the friction cones 40 move inwards. This will allow the worm wheel 36 to rotate.

Figure 5 shows an alternative design of the cartridge exchange brake utilizing a locking disc 46 arranged with splines 48, which splines cooperate with corresponding splines in a seat 50. In this case, in order to release the worm wheel 36 for resetting the device, a protruding end of a shaft 52 on which the worm wheel 36 is attached comes in contact with a part of the device, e.g. a lid, whereby the locking disc 46 is moved out of contact with the seat 50 and the worm wheel is free to rotate.

When the lid subsequently is closed, the worm wheel 36 is again locked from rotation.

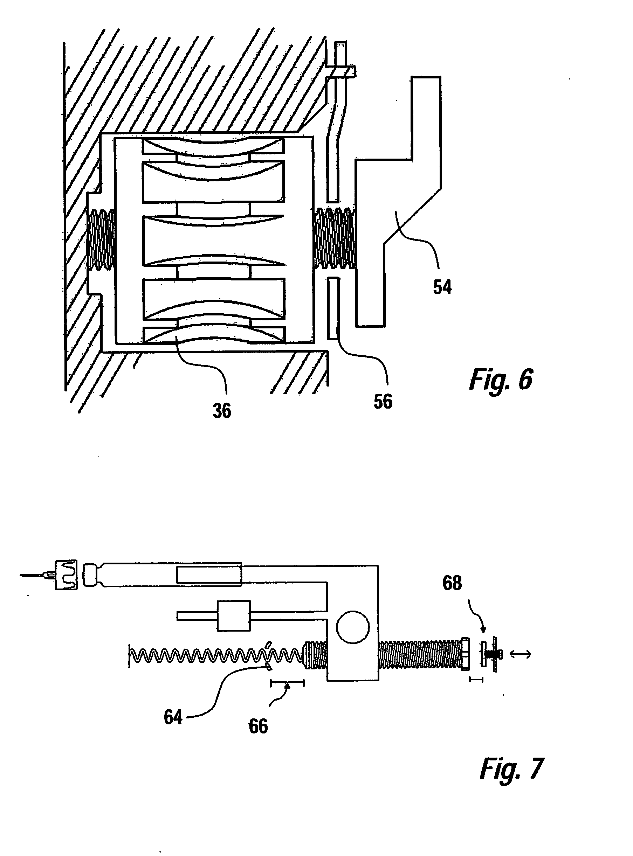

Figure 6 shows another and perhaps more robust variant of the cartridge exchange brake using friction as locking method. Here the worm wheel 36 will be locked by friction from a locking screw 54 which applies pressure on a locking washer 56. The locking screw 54 may be arranged such that it is rotated when the device is opened for cartridge exchange, for example via the lid with an inter-connecting mechanism that rotates the locking screw when the lid is opened and closed.

As is evident from the above description, the presented brake embodiments may be used in other applications, i.e. the brake solutions for the cartridge exchange may be utilised, perhaps with some modification, as dose release brake and vice versa. It is also to be understood that there may be other applicable brake solutions that may perform the desired functions.

### Dose setting unit

The device further preferably comprises a dose setting unit 60, which is operationally connected to the plunger rod 12 via a transversal support member 58, hereafter named plunger rod housing, to which the plunger rod 12 and the dose release brake rod 22 are attached. The dose setting unit comprises the threaded dose setting screw 38 that is threadidly engaged with corresponding threads in a passage through the plunger rod housing 58. The distal end of the dose setting screw 38 is arranged with a dose setting knob 62, which may be operated manually by a user such that the dose setting screw is moved in the distal direction, whereby the proximal end of the dose setting knob is moved out of contact with a fixed stop surface 64. The distance between the proximal end of the dose setting knob and the stop surface corresponds to a certain dose quantity of medicament, indicated by 66 in Fig. 7.

The size of the dose may be shown either in a digital or an analogue way depending on the configuration of the device. The mechanism does not allow user to set a larger dose than what is left in the cartridge.

There is also a possibility to apply a feature, if required, to adjust the maximum set dose mechanically. This can for instance be done by authorized personnel with a screwdriver like special instrument by adjusting a dose stop detail 68.

### Injection force means

The dose setting screw 38 is connected to an injection force means such as a drive spring 14 such that the drive spring urges dose setting screw 38 to perform a linear movement in the proximal direction, as well as the plunger rod 12, via the plunger rod housing 58 when the dose release brake 10 is operated to release the dose release brake rod 22. This causes the stopper 16 to be moved in the proximal direction by the plunger rod 12, delivering medicament through the medicament delivery member 20, until the proximal end of the dose setting screw is moved in contact with the stop surface 64.

The injection force means 14 is preferably a spring force member that is tensioned when the dose is set, i.e. a compression spring that is extended when the dose setting screw 38 is moved in the distal direction. The advantage with this configuration is that the spring is provided with a pre-tension, that may be rather low, when the dose setting screw is in contact with the stop surface, and is tensioned more when a dose is set. This reduces the "resting force", i.e. the force on the device when it is not used.

However, other types of injection force means may be utilised for performing a linear movement of the plunger rod, such as spiral springs that are compressed during setting of dose, gas springs, clock springs, and the like. Also if the device is to deliver only one dose from a medicament container, the dose setting screw 38 may be omitted. Instead the injection force may be acting directly on e.g. the plunger rod housing 58, which is held in the tensioned state by the dose release brake 10.

The above mentioned components and functions can be regarded as a platform, to which additional components and functions may be added depending on the specific application or specific requests from users and customers. These may for example comprise an automatic penetration function, a priming function, a needle covering feature after completed injection, mechanical and/or electronic dose information means, just to mention a few.

### Non-limiting example of a medicament delivery device

A non-limiting example of the present invention will now be described in connection with the drawings 8 - 15. It comprises a medicament delivery device 70, in the shown embodiment an injector, for medicament delivery. It comprises a housing generally elongated, in the embodiment comprising three parts; a central part 72 and two side parts 74, a b. It should however be understood that the housing could be designed in many other ways without departing from the scope of the invention.

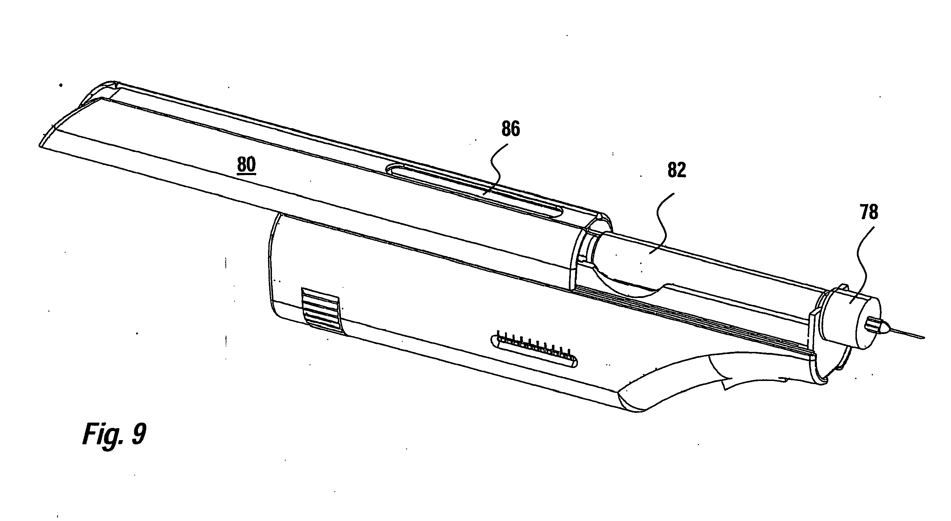

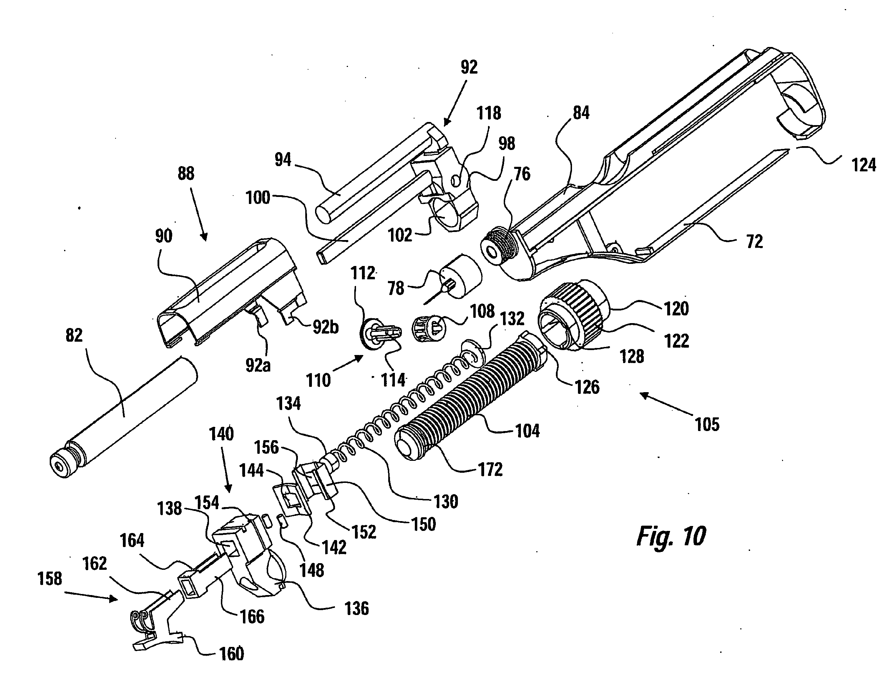

At the proximal end of the housing a threaded neck 76 is arranged, onto which a medicament delivery member, such as an injection needle 78, is releasibly attached. It is however to be understood that other types of medicament delivery members could be used such as mouthpieces, nozzles, nebulizers and the like. The device is further arranged with a lid 80 which is arranged slidable in relation to the housing, as seen in Figure 9. When the lid is in the opened position a user is capable of placing a medicament container 82 inside the device in a holder 84 arranged in the central part 72 of the housing. The lid 80 is further arranged with an opening or a window 86 through which the medicament container 82 and its content is visible. Attached to, or made integral with, the lid is a cartridge exchange mechanism 88 comprising a cover part 90 arranged with two downwardly pointing arms 92 a, b attached to the distal area of the cover 90, Fig. 10.

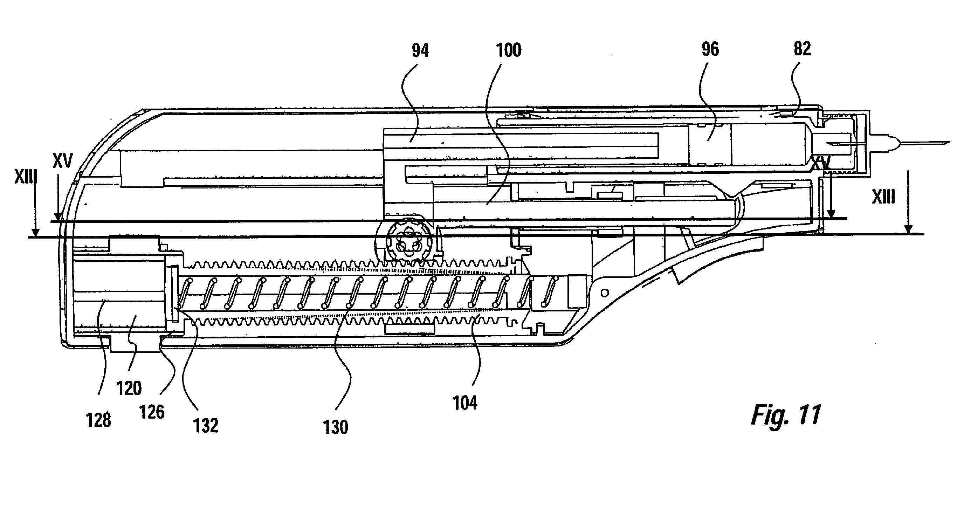

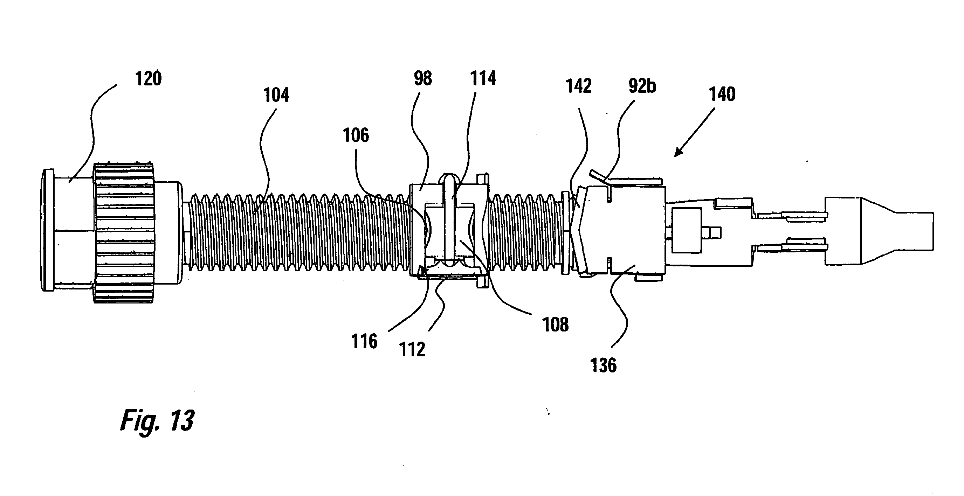

A drive unit 92 is further provided in the housing, Fig. 10. It comprises an elongated plunger rod 94 arranged to act on a movable stopper 96, Fig. 11, placed inside said medicament container. The plunger rod 94 is further attached to or made integral with, a transversally extending drive member 98. To the drive member 98 a rod 100 is attached, or made integral with, which rod hereafter is named dose release brake rod. The dose release brake rod 100 has a generally rectangular cross-section and extends generally parallel with the plunger rod 94. The drive member 98 is further arranged with a generally circular passage 102 extending generally parallel with the plunger rod 94. Through the passage 102 a threaded dose setting screw 104 of a dose setting mechanism 105 is arranged. Further a second passage 106, Fig. 13, is arranged in the drive member extending generally perpendicular to the first passage and positioned such that there is an opening between the two passages.

In the second passage 106 a worm wheel 108 is positioned with its threads engaging the threads of the dose setting screw. To the worm wheel a locking member 110 is arranged, comprising a friction disc 112 having a central shaft 114 that engages the worm wheel such that a rotational lock is obtained between the two but allowing a relative axial movement. The friction disc 112 has a conical circumferential surface that is capable of cooperating with a conical circumferential surface 116 of the second passage 106. The two conical surfaces are preferably arranged with friction increasing surfaces such as indentations, grooves, ribs or the like. Further the end of the central shaft 114 of the locking member protrudes somewhat through a side surface of the drive member via a passage 118 when the conical surfaces are in engagement with each other. Further a spring (not shown) is arranged for urging the conical surfaces in engagement.

The dose setting mechanism 105 further comprises a dose setting knob 120 having a generally tubular form arranged rotational in the housing having a circumferential surface 122 protruding through an opening 124 in the housing for manual rotation of the dose setting knob 120. The distal end of the dose setting rod 104 is arranged to protrude into the interior of the dose setting knob 120 and is provided with a number of longitudinally extending ribs 126 in the distal area, which ribs fit into corresponding longitudinally extending grooves 128 on the inner surface of the dose setting knob, thus providing a rotational lock and a longitudinal movement between the dose setting knob 120 and the dose setting screw 104.

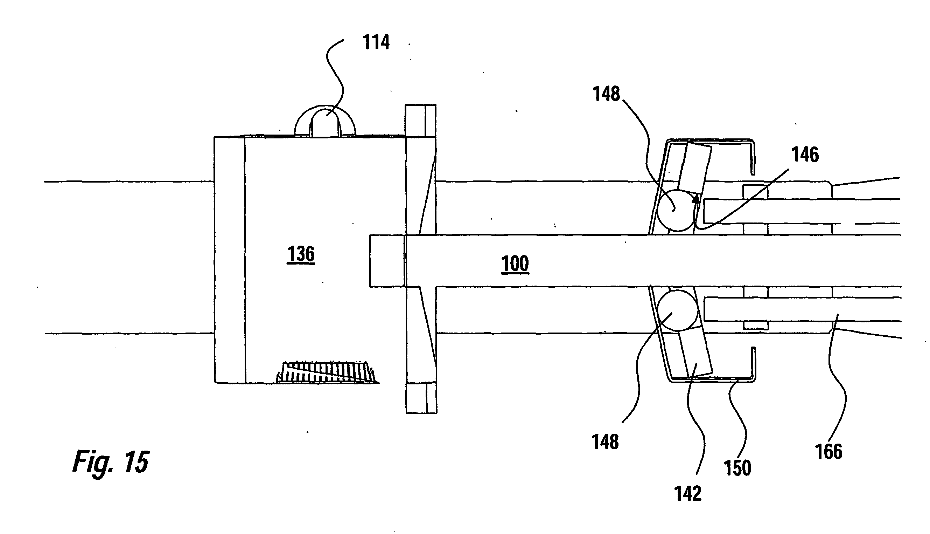

Inside the dose setting screw 104 a drive spring 130 is arranged. Its distal end is attached to a plate 132, which in turn is seated in a circumferential groove on the inner surface in the distal area of the dose setting screw 104. The proximal end of the drive spring 130 is extending through the proximal end of the dose setting screw 104 and is attached to a plate 134 which is seated in a fixture arranged in a post 136 in turn fixedly attached to the housing. Further the dose release brake rod 100 extends through a passage 138 arranged in the post, which passage 138 generally has the same cross-sectional shape as the dose release brake rod. A dose release brake 140 is further arranged to the post. It comprises a washer 142 that is positioned adjacent the passage on its distal side having a rectangular hole 144. The washer 142 has a somewhat V-shape as seen from above, Fig. 15, thereby creating inclined side surfaces 146 of the rectangular hole 144. Two rolls 148 are placed between the side surface of the dose release brake rod 100 and the inclined side surfaces 146 of the washer 142. The washer 142 and the rolls 148 are held in position in relation to the post 136 by a holder 150, preferably of sheet metal, placed in the distal direction and having a rectangular opening through which the dose release brake rod 100 protrudes, and being attached to the post by inwardly formed edges 152 fitting into grooves 154 on side surfaces of the post 136. Adjacent the opening in the holder two flexible tongues 156 are arranged in the holder, which tongues push on the rolls 148 to urge them in the proximal direction, whereby they are forced against the inclined surfaces 146 and thus towards the side surfaces of the dose release brake rod 100, thereby locking the dose release brake rod 100 from movement in the proximal direction.

A dose release mechanism 158 is arranged adjacent the post and the dose release brake. It comprises an activation button 160 pivotally attached to and accessible via an opening in the housing close to the proximal part of the device. The activation button is provided with two arms 162 pointing in the distal direction and positioned on either side of the dose release brake rod 100. The distal ends of the arms 162 are in contact with a rectangularly shaped sleeve 164 arranged slidable around the dose release brake rod 100. The sleeve is further arranged with two distally directed arms 166 on either side of the dose release brake rod 100 and extending into the passage 138 of the post 136. The distal ends of the arms 166 of the sleeve 164 are positioned adjacent the rolls 148.

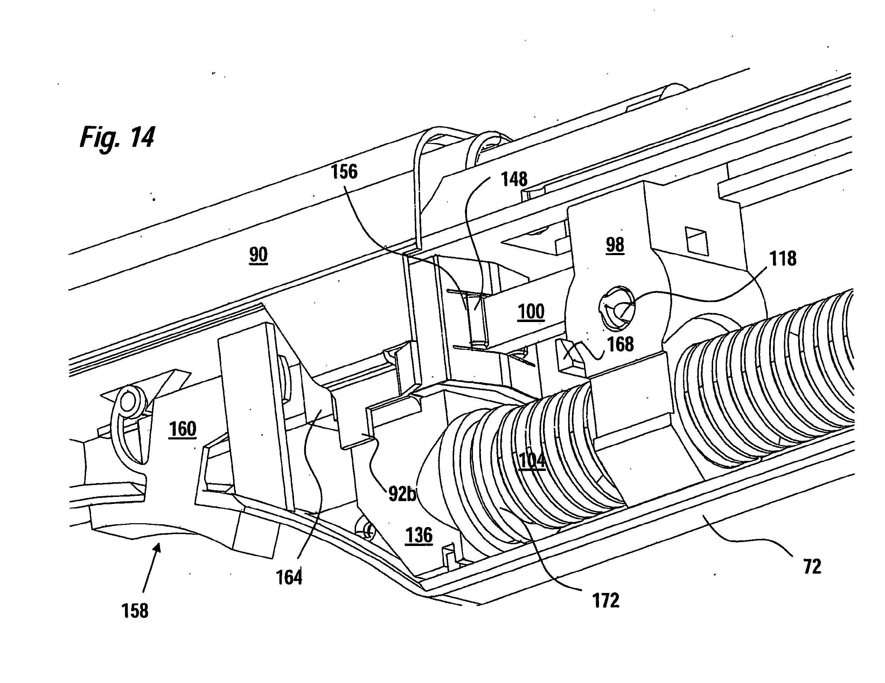

The device is intended to function as follows. When the device is delivered to a user, it is normally not loaded with a medicament cartridge. Thus in order to insert a medicament cartridge 82 the lid 80 is slid in the distal direction in relation to the housing, whereby the medicament holder 84 for housing the medicament cartridge 82 is accessible. The movement of the lid also moves the cartridge exchange mechanism 88 in the distal direction whereby the arm 92b comes in contact with the protruding end of the central shaft 114 of the locking member whereby the friction disc 112 is moved out of contact with the seat, which in turn releases the worm wheel. Further the distally directed side edges of the arms 92a, 92b come in contact with abutments 168 on the drive member 98, Fig. 14, which forces the drive member to move in the distal direction while the worm wheel rotates along the dose setting screw. The drive member is not prevented from moving in the distal direction by the dose release brake 140, since the rolls 148 allow movement of the dose release brake rod in that direction but not in the opposite, proximal, direction.

When the medicament container now is placed in the holder 84, the lid 80 is slid back to its closed position, whereby the cartridge exchange mechanism is also moved in the proximal direction. The shaft 114 is then released and the friction disc 112 is again moved in contact with the seat, thereby again locking the worm wheel against rotation.

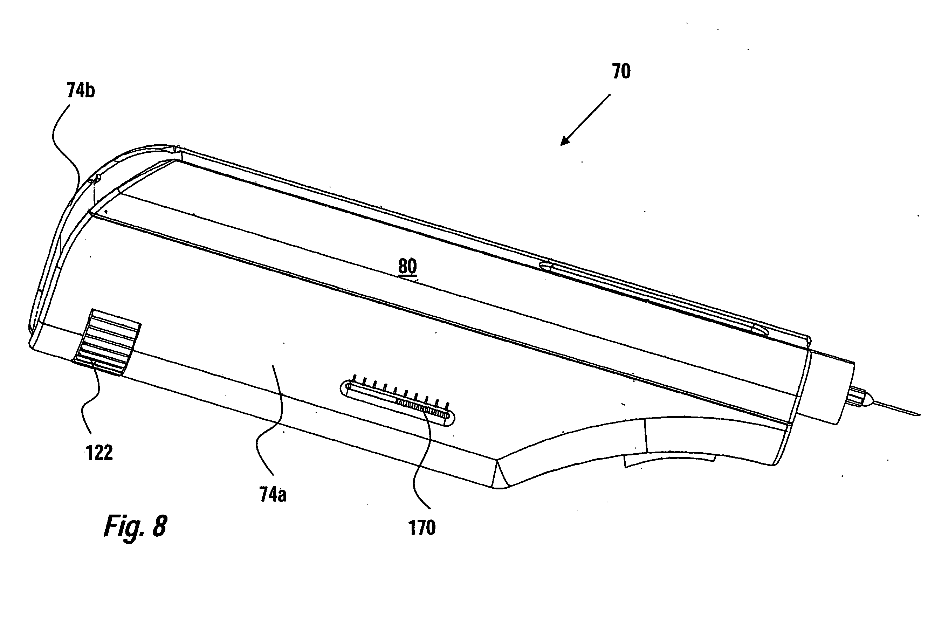

The user is now to set a dose of medicament. He/she then turns the dose setting knob 120 whereby the dose setting screw 104 is rotated. Due to the engagement with the locked worm wheel, the rotation of the dose setting screw causes it to move in the distal direction. The distal movement of the dose setting screw causes the spring to be tensioned due to that it is pulled in the longitudinal direction since its proximal end is fixed to the post 136 and its distal end is fixed in relation to the distal end of the dose setting screw. When a desired or prescribed dose is set, which may be viewed in a dose window 170, Fig. 8, the device is ready for an injection.

The user now places the device at the dose delivery site and in the case of an injector an injection needle 78 penetrates the injection site. The actuation button 160 is now depressed whereby it is pivoted and its arms 162 are moved in the distal direction. This movement causes them to come in contact and move the sleeve 164 whereby its arms 166 act on the rolls 148 of the dose release brake 140, which rolls are moved out of locking contact with the dose release brake rod, against the force of the tongues 156. The release of the dose release brake rod means that the drive member, the dose setting screw and the plunger rod are free to move and they will now move in the proximal direction due to the force of the spring 130, which causes an injection of medicament from the medicament container because the plunger rod forces the stopper in the proximal direction. The complete dose is delivered when the proximal end of the dose setting screw is moved in contact with a distally directed surface of the post 136. The user may now remove the device from the medicament delivery site.

Should the user however feel uncomfortable or feel pain during the injection sequence, he/she may pause the sequence by merely releasing the actuation button 160. This causes the rolls to be able to be moved in the proximal direction again due to the force of the tongues, and due to the inclined surfaces the rolls are pressed against the dose release brake rod, whereby the movement of the dose release brake rod, the drive member, the dose setting screw and the plunger rod is stopped. After a pause, the injection sequence may be continued by again pressing the actuation button.

When an injection has been performed the used medicament delivery member may be discarded in a safe way. When a subsequent medicament delivery is to be performed a new medicament delivery member is attached and a dose is set as described previously. However, the device is designed such that it is not possible to set a larger dose than is remaining in the container in that the proximal part of the dose setting screw is arranged with a circumferential ledge 172 and when this ledge reaches the worm wheel 108, it is no longer possible to screw the dose setting screw 104 any further in the distal direction. The quantity of the last remaining dose is visible in the dose window 170, whereby it is possible for the user to complete the required injected dose with an additional subsequent injection of the missing dose quantity.

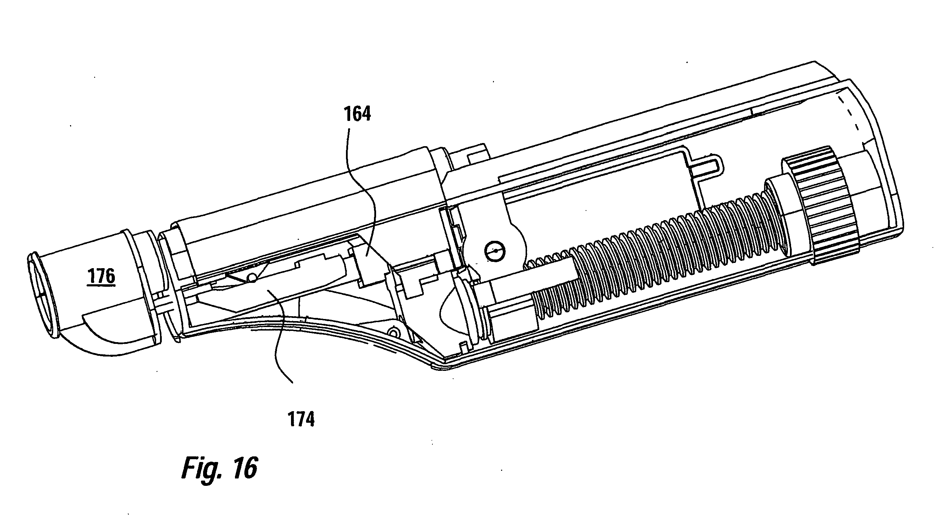

An alternative to a dose release mechanism is shown in figure 16. The mechanism comprises an arm 174, hereafter named needle shield arm, is arranged slidable in the proximal part of the housing. The needle shield arm 174 extends through the proximal end of the device and is attached to a needle shield 176, which needle shield has a generally tubular shape.

With this dose release mechanism, the user presses the front end with the needle shield 176 against the injection site whereby a penetration is performed. Also the needle shield 176 with its needle shield arm 174 is moved axially. The needle shield arm presses on the sleeve 164 whereby its arms 166 act on the rolls 148 of the dose release brake 140, which rolls are moved out of locking contact with the dose release brake rod 100, against the force of the tongues 156. This in turn enables the dose release brake rod 100 to be released whereby the whole drive unit with the plunger rod 94 is moved forward by the force of the injection spring 130. The movement of the plunger rod 94 causes a displacement of the stopper 96 whereby a dose of medicament is injected through the needle. The injection sequence is ended when the dose setting screw comes in contact with the stop surface of the post 136.

It is to be understood that the embodiments of the invention described above and shown in the drawings are to be regarded only as non-limiting examples of the invention and that it may be modified in many ways within the scope of the patent claims.

## Claims

1. Medicament delivery device comprising
- a housing (72, 74a, b);
- a medicament container (82) arranged inside said housing (72, 74a, b),
- a drive mechanism (94, 118, 104, 130) comprising a plunger rod (94) capable of - acting on a stopper (96) inside said medicament container (82) for expelling a dose of medicament,
- said drive mechanism (94, 118, 104, 1-30) further comprising a force member (130) acting on said plunger rod,
- an actuation mechanism (140, 158) capable of releasing said drive mechanism upon activation,
- that said actuation mechanism (140, 158) comprises a locking mechanism (140) capable of releasibly locking said plunger rod (94) in any positions between an initial start position and an end position, wherein said locking mechanism (140) comprises a one-way locking unit enabling the plunger rod to move in only a proximal direction,
**characterised in that** said locking unit is arranged to act on a further rod (100) arranged generally parallel to said plunger rod (94).

2. Medicament delivery device according to any of the preceding claims,
wherein it further comprises a dose setting mechanism (105).

3. Medicament delivery device according to claim 2, wherein said one-way locking unit comprises rolls or balls (148) in contact with said plunger rod and a locking member (142) arranged with inclined surfaces (146) acting on said rolls or balls (148).

4. Medicament delivery device according to any of the preceding claims,
wherein said actuation mechanism is activated by a manually operated actuation button (160).

5. Medicament delivery device according to any of the preceding claims,
wherein said actuation mechanism is activated by a needle shield (176) arranged in the proximal end of said medicament delivery device.

6. Medicament delivery device according to claim 2, wherein said dose setting mechanism comprises a threaded dose setting member (104) and wherein it further comprises second locking means (110) for releasibly locking said threaded dose setting member (104).

7. Medicament delivery device according to claim 6, wherein said second locking means (110) is manually activated to release said threaded dose setting member (104) when a medicament container (88) is to be placed in said medicament delivery device.

## Patentansprüche

1. Medikamentenabgabevorrichtung, umfassend:
- ein Gehäuse (72, 74a, b);
- einen Medikamentenbehälter (82), der innerhalb des Gehäuses (72, 74a, b) angeordnet ist;
- einen Antriebsmechanismus (94, 118, 104, 130), der in eine Stößelstange (94) aufweist, die in der Lage ist, auf einen Stopper (96) innerhalb des Medikamentenbehälters (82) zu wirken, um eine Medikamentendosis auszuwerfen,
- wobei der Antriebsmechanismus (94, 118, 104, 130) ferner ein auf die Stößelstange wirkendes Kraftelement (130) aufweist,
- einen Betätigungsmechanismus (140, 158), der in der Lage ist, bei Aktivierung den Antriebsmechanismus zu lösen,
- dass der Betätigungsmechanismus (140, 158) einen Sperrmechanismus (140) aufweist, der in der Lage ist, die Stößelstange (94) in beliebigen Stellungen zwischen einer Anfangsstartposition und einer Endposition lösbar zu arretieren, und worin der Sperrmechanismus (140) eine Einweg-Sperreinheit aufweist, die in der Lage ist, die Stößelstange nur in einer proximalen Richtung zu bewegen,
**dadurch gekennzeichnet, dass** die Sperreinheit angeordnet ist, um auf eine weitere Stange (100) zu wirken, die allgemein parallel zu der Stößelstange (94) angeordnet ist.

2. Medikamentenausgabevorrichtung nach einem der vorhergehenden Ansprüche, worin sie ferner einen Dosiseinstellmechanismus (105) aufweist.

3. Medikamentenausgabevorrichtung nach Anspruch 2, worin die Einweg-Sperreinheit Rollen oder Kugeln (148) in Kontakt mit der Stößelstange sowie ein Sperrelement (142) aufweist, das mit geneigten Oberflächen (146) angeordnet ist, die auf die Rollen oder Kugeln (148) wirken.

4. Medikamentenausgabevorrichtung nach einem der vorhergehenden Ansprüche, worin der Betätigungsmechanismus durch einen manuell betätigten Betätigungsknopf (160) aktiviert wird.

5. Medikamentenausgabevorrichtung nach einem der vorhergehenden Ansprüche, worin der Betätigungsmechanismus durch ein im proximalen Ende der Medikamentenausgabevorrichtung angeordnetes Nadelschild (176) aktiviert wird.

6. Medikamentenausgabevorrichtung nach Anspruch 2, worin der Dosiseinstellmechanismus ein Dosiseinstell-Gewindeelement (104) aufweist, und worin sie ferner ein zweites Sperrmittel (110) zum lösbaren Arretieren des Dosiseinstell-Gewindeelements (104) aufweist.

7. Medikamentenausgabevorrichtung nach Anspruch 6, worin das zweite Sperrmittel (110) manuell aktiviert wird, um das Dosiseinstell-Gewindeelement (104) zu lösen, wenn ein Medikamentenbehälter (88) in der Medikamentenausgabevorrichtung angeordnet werden soll.

## Revendications

1. Dispositif d'administration de médicament comprenant
- un boîtier (72, 74a, b) ;
- un récipient de médicament (82) agencé à l'intérieur dudit boitier (72, 74a, b),
- un mécanisme d'entraînement (94, 118, 104, 130) comprenant une tige de piston (94) apte à agir sur un piston (96) à l'intérieur dudit récipient de médicament (82) pour expulser une dose de médicament,
- ledit mécanisme d'entraînement (94, 118, 104, 130) comprenant en outre un organe de force (130) agissant sur ladite tige de piston,
- un mécanisme d'actionnement (140, 158) apte à libérer ledit mécanisme d'entraînement lors de l'activation,
- ledit mécanisme d'actionnement (140, 158) comprend un mécanisme de verrouillage (140) apte à verrouiller de manière libérable ladite tige du piston (94) en toutes positions entre une position de départ initiale et une position finale, ledit mécanisme de verrouillage (140) comprenant une unité de verrouillage unidirectionnel permettant à la tige du piston de se déplacer uniquement dans une direction proximale,
**caractérisé en ce que** ladite unité de verrouillage est agencée pour agir sur une tige supplémentaire (100) agencée d'une manière généralement parallèle à ladite tige de piston (94).

2. Dispositif d'administration de médicament selon l'une des revendications précédentes, qui comprend en outre un mécanisme (105) de réglage de dose.

3. Dispositif d'administration de médicament selon la revendication 2, dans lequel ladite unité de verrouillage unidirectionnel comporte des rouleaux ou des billes (148) en contact avec ladite tige de piston et un organe de verrouillage (142) agencé avec des surfaces inclinées (146) agissant sur ledit rouleaux ou billes (148).

4. Dispositif d'administration de médicament selon l'une des revendications précédentes, dans lequel ledit mécanisme d'actionnement est activé par un bouton de commande (160) à actionnement manuel.

5. Dispositif d'administration de médicament selon l'une des revendications précédentes, dans lequel ledit mécanisme d'actionnement est activé par une pièce (176) de protection de l'aiguille disposée sur l'extrémité proximale dudit dispositif de livraison médicament.

6. Dispositif d'administration de médicament selon la revendication 2, dans lequel ledit mécanisme de réglage de dose comprend un organe fileté (104) de réglage de dose, le dispositif comprenant en outre des deuxièmes moyens de verrouillage (110) pour le verrouillage libérable dudit organe fileté de réglage de dose (104).

7. Dispositif d'administration de médicament selon la revendication 6, dans lequel ledit deuxième moyen de verrouillage (110) est activé manuellement pour libérer ledit organe fileté (104) de réglage de dose lorsqu'un conteneur (88) de médicament doit être placé dans ledit dispositif d'administration de médicament.
